Europäisches Patentamt

European Patent Office

Office européen des brevets

(19)

(11) Numéro de publication: **0 249 514**
**B1**

(12) **FASCICULE DE BREVET EUROPEEN**

(45) Date de publication du fascicule du brevet: **04.04.90**

(21) Numéro de dépôt: **87401063.0**

(22) Date de dépôt: **11.05.87**

(51) Int. Cl.⁵: **G 01 N 33/28**

(54) Procédé de caractérisation d'un catalyseur par la détermination de ses activités de conversion et de cokéfaction.

(30) Priorité: **27.05.86 FR 8607690**

(43) Date de publication de la demande:
**16.12.87 Bulletin 87/51**

(45) Mention de la délivrance du brevet:
**04.04.90 Bulletin 90/14**

(84) Etats contractants désignés:
**BE DE ES GB IT NL**

(56) Documents cités:
**EP-A-0 026 012**
**US-A-4 099 923**
**US-A-4 568 426**

(73) Titulaire: **INSTITUT FRANCAIS DU PETROLE**
**4, Avenue de Bois-Préau**
**F-92502 Rueil-Malmaison (FR)**

(72) Inventeur: **Toulhoat, Hervé**
**51, avenue Charles Tellier**
**F-78800 Houilles (FR)**
Inventeur: **Favre, Anne**
**30, Boulevard Robespierre**
**F-78300 Poissy (FR)**

Courier Press, Leamington Spa, England.

# EP 0 249 514 B1

**Description**

La présente invention concerne un procédé de caractérisation de catalyseur par la détermination de ses activités de conversion et de cokéfaktion. Plus particulièrement, le procédé permet la caractérisation de catalyseurs de raffinage, de résidus de pétrole notamment, ces catalyseurs pouvant être neufs ou usés.

Dans le domaine du raffinage du pétrole, les catalyseurs présentent de manière préférée une activité plus ou moins convertissante qui, au cours de l'usage, a tendance à s'atténuer et à devenir de plus en plus cokéfiante.

Ces catalyseurs peuvent se ranger en deux groupes, le premier concernant le domaine du craquage catalytique et le second le domaine de l'hydrotraitement.

Pour caractériser les catalyseurs de craquage, il est connu d'utiliser le test dit M.A.T. (Micro Activity Test) décrit dans la norme ASTM D39-07. Cette méthode est longue car elle implique pour obtenir un bilan, le passage par une distillation simulée sur colonne chromatographique et une analyse ex-situ du coke résiduel sur un analyseur de carbone, ce qui la rend difficilement reproductible.

Par ailleurs, pour caractériser les catalyseurs d'hydrotraitement on met en oeuvre des tests sous haute pression en présence d'hydrogène suivis d'analyses élémentaires impliquant un coût très elévé et une durée totale d'expérience atteignant couramment une quinzaine de jours.

Les informations obtenues de ces tests longs et coûteux permettent de qualifier l'activité plus ou moins convertissante et parallèlement plus ou moins cokéfiante des catalyseurs mis en oeuvre.

L'art antérieur est décrit particulièrement par les brevets suivants: US—A—4 568 426, EP—A— 0 026 012 et US—A—4 099 923.

Le brevet US—A—4 568 426 décrit un appareil sophistiqué susceptible de reproduire avec précision et simplicité la mesure du carbone Conradson (CCR) où l'échantillon est chauffé en atmosphère inerte, les produits gazeux sont purgés en atmosphère inerte et le résidu est pesé.

Le brevet EP—A—0 026 012 décrit une méthode de détermination de la densité API d'une huile par vaporisation des composés volatils et pyrolysables et mesure de la quantité d'hydrocarbures dans une gamme de température par exemple de 350 à 750°C par rapport à la quantité totale vaporisée.

Enfin, le brevet US—A—4 099 923 décrit une unité de présélection de catalyseurs en présence d'hydrocarbures seuls ou en présence d'ammoniac ou d'air ou des deux gaz. Une partie aliquote des gaz de réaction est séparée des liquides et solides et est transférée dans un chromatographe en phase gazeuse où elle est analysée.

Un des objets de l'invention est donc de mettre au point un procédé qui permette à partir d'informations n'étant pas nécessairement les mêmes que celles selon l'art antérieur, de caractériser des catalyseurs de manière rapide, simple, bon marché, et de manière intégrée d'où répétable.

Un autre objet de l'invention est de déterminer à partir d'une charge de référence l'activité convertissante des catalyseurs par l'étude des répartitions des produits légers et des produits de pyrolyse obtenus ainsi que leur activité cokéfiante, c'est-à-dire leur potentiel de désactivation par cokage par la répartition des produits de pyrolyse et de cokéfaction de cette charge. En d'autres termes, il s'agit de classifier de manière rapide des matériaux catalytiques en relation avec le niveau de conversion et de cokage des produits lourds.

Un autre objet est de savoir comment se comporte un catalyseur donné vis-à-vis de charges différentes et donc de prédire le potentiel de volarisation économique de ces charges.

Un autre objet de l'invention est aussi d'avoir la possibilité d'ajuster rapidement les propriétés du catalyseur aux propriétés de la charge.

Le procédé selon l'invention concerne donc la caractérisation d'un catalyseur par la détermination de ses activités de conversion et de cokéfaction. Il comprend les étapes successives suivantes:

a) on imprègne le catalyseur par un mélange d'hydrocarbures (ou charge),

b) un chauffe sous flux de gaz inerte le catalyseur imprégné jusqu'à une température $\theta_2$ comprise entre 300°C et 380°C de façon à réaliser durant le temps de montée à ladite température une étape de conversion—évaporation et l'on produit un effluent de conversion-évaporation,

c) on mesure sur ledit effluent de conversion-évaporation, au cours du chauffage, au moyen d'un détecteur d'hydrocarbures, un signal Q représentatif des hydrocarbures générés durant ladite étape de conversion-évaporation,

d) on poursuit le chauffage depuis la température $\theta_2$ jusqu'à la température $\theta_3$ comprise entre 500°C et 1000°C de manière progressivement croissante et sous flux de gaz inerte, de façon à réaliser durant la temps de montée à ladite température une étape de pyrolyse et l'on produit un effluent de pyrolyse et un résidu,

e) on mesure sur ledit effluent de pyrolyse, au cours du chauffage, au moyen d'un détecteur d'hydrocarbures, un signal P représentatif des hydrocarbures pyrolysés générés durant ladite étape de pyrolyse,

f) On réaliser ensuite sur ledit résidu une combustion à une température $\theta_4$ comprise entre 500°C et 1500°C sous courant de gaz oxydant, ladite combustion produisant un effluent gazeux de combustion,

g) on mesure sur ledit effluent gazeux de combustion, au moyen d'au moins un détecteur approprié, un signal R représentatif de la quantité dudit résidu, et

h) on déduit à partir desdites mesures des signaux Q, P, et R les activités de conversion et de cokéfaction dudit catalyseur.

On opèrera généralement en présence d'une même charge pour l'étude comparative de différents catalyseurs, ou en présence d'un même catalyseur pour l'étude comparative de différentes charges, toutes conditions opératoires restant identiques par ailleurs.

Par mélange d'hydrocarbures, on entend selon l'invention des composés comprenant du carbone et de l'hydrogène mais pouvant également comprendre de l'oxygène, du soufre et de l'azote. Le mélange d'hydrocarbures correspond par exemple à des coupes de point d'ébullition initial, à pression atmosphérique normale, qu moins égal à 350°C. On pourra tolérer dans le mélange une faible proportion d'hydrocarbures de point d'ébullition inférieur à 350°C, bien que ce ne soit pas préférable.

Les catalyseurs concernés sont par exemple ceux mis en oeuvre dans les procédés de raffinage, notamment ceux utilisés dans le cadre du craquage catalytique ou bien ceux utilisés dans les hydrotraitements.

On peut selon un mode de mise en oeuvre particulier du procédé, mesurer, au moyen d'un détecteur spécifique du dioxyde de carbone, un signal représentatif d'une quantité de dioxyde de carbone résultant de la combustion et par conséquent, de la quantité du résidu. Mais s'il est utile de tenir compte, après l'étape de combustion, de la présence d'oxydes de soufre et/ou d'azote pour l'étude d'une charge à forte teneur en soufre et/ou azote, on peut combiner en série plusieurs détecteurs appropriés à ces oxydes ou utiliser un seul détecteur multicanal à infrarouge par exemple pour obtenir un signal R représentatif des quantités de dioxyde de carbone, d'oxyde de soufre et d'oxyde d'azote et donc de la quantité dudit résidu.

Avant l'étape de chauffage, le catalyseur est imprégné en totalité par le mélange d'hydrocarbures. On opère de manière aussi homogène que possible, de préférence en solution dans un solvant de point d'ébullition très inférieur au point d'ébullition initial du mélange d'hydrocarbures de façon qu'il puisse être éliminé sans entraîner tout ou partie du mélange. Avantageusement on peut opérer en présence d'un solvant aromatique léger, de préférence un alkyl benzène pour pouvoir dissoudre les asphaltènes. La concentration en mélange d'hydrocarbures peut être comprise par exemple entre 1 et 30% et de préférence entre 10 et 20%. La porosité de la matrice catalytique est remplie de la solution ainsi réalisée et on procède ensuite à une élimination du solvant par des moyens connus.

L'invention sera mieux comprise au vu des figures et exemples illustrant de façon non limitative la mise en oeuvre du procédé, parmi lesquels:

— la figure 1 illustre schématiquement les moyens de mise en oeuvre du procédé,

— la figure 2 représente le diagramme ternaire où sont portés les indices, $F_R$, $F_2$ et $F_1$ déterminés ci-dessous,

— les figures 3 et 4 montrent les corrélations de résultats relatifs à des catalyseurs d'hydrotraitement, selon le procédé de l'invention et selon l'art antérieur, et

— les figures 5 et 6 sont relatives à des catalyseurs de craquage catalytique et montrent des corrélations entre des résultats selon l'invention et ceux selon l'art antérieur (M.A.T.).

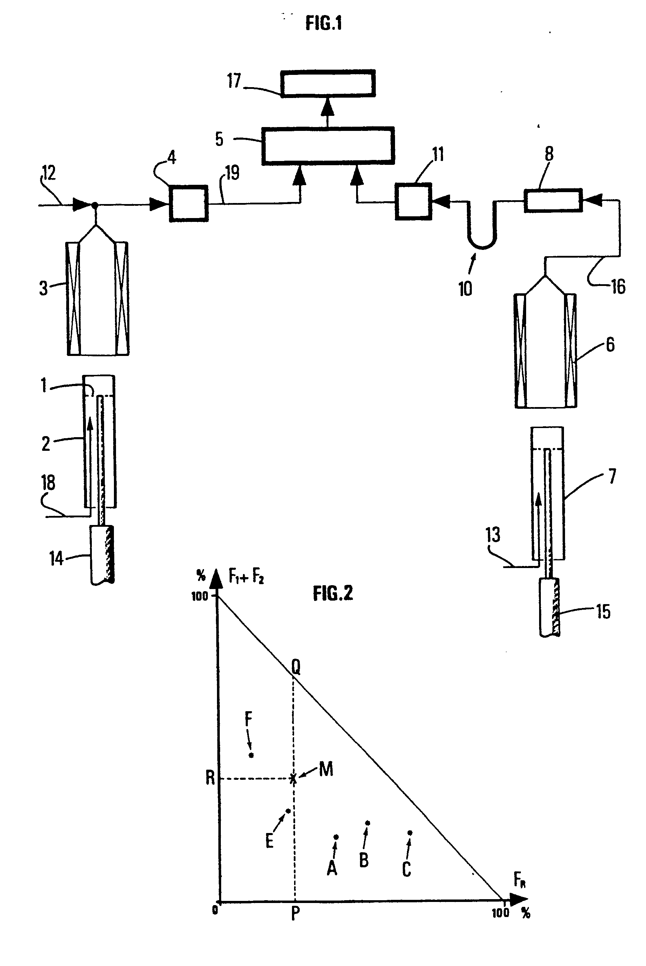

Selon la figure 1 représentant un mode de réalisation particulier de l'invention, le catalyseur une fois imprégné par l'échantillon d'hydrocarbures est broyé, puis introduit dans une nacelle 1 prenant place au sommet d'un piston 2 qui est mis en mouvement par un jeu de vérins 14. La nacelle 1 est placée dans un premier four 3 dans lequel on effectue grâce à une alimentation 18 en gaz inerte une étape de conversion-évaporation par chauffage sous flux de gaz inerte, par exemple azote, argon on hélium et de préférence de l'hélium pour avoir une meillure réponse du détecteur 4.

Le débit spécifique de gaz inerte rapporté à la quantité de matrice poreuse imprégnée est par exemple de 1 à 50 millilitres par milligramme et par minute, et de préférence de 2 à 10 ml/mg/min.

Le chauffage sous flux de gaz inerte peut s'effectuer de la manière suivante:

1) un isotherme pouvant aller jusqu'à 20 min et de préférence jusqu'à 5 min à une température $\theta_1$ comprise entre la température ambiante et celle due à l'inertie thermique du four une fois refroidi, cette température $\theta_1$ étant au moins égale à la température ambiante puis une programmation de température avec une vitesse de montée en température $V_1$ comprise entre 2°C/min et 50°C/s jusqu'à une température du four $\theta_2$ comprise entre 300°C et 380°C, ou bien,

2) une montée en température, de la température ambiante jusqu'à la température $\theta_2$, comprise entre 2°C/min et 50°C/s ou bien,

3) une montée en température sensiblement instantanée de l'échantillon lorsqu'il est mis directement, à partir de la température ambiante où il se trouve à l'état initial, dans le four chauffé à une température $\theta_2$ définie ci-dessus.

Les effluents de l'étape de conversion-évaporation sont véhiculés par une ligne 19 alimentée en gaz inerte 12 et détectés au cours du temps par un détecteur d'hydrocarbures, par exemple à ionisation de flamme (FID) 4 préalablement étalonné avec un échantillon de référence. On peut n'effectuer qu'une seule mesure du signal Q ou faire une série de mesures; dans ce cas les résultats sont intégrés par tranche (par exemple une intégration toutes les 1 à 120 secondes, de préférence toutes les 40 à 120 secondes) et conservés en mémoire par le microprocesseur 5. On obtient ainsi un signal Q en fonction du temps et/ou de la température représentatif des hydrocarbures générés durant cette étape.

On effectue ensuite une pyrolyse, toujours sous flux de gaz inerte grâce à une programmation de température de $\theta_2$ à $\theta_3$, la température $\theta_3$ étant supérieure à $\theta_2$ et comprise entre 500°C et 1000°C, de préférence entre 600°C et 700°C et à une vitesse comprise entre 2°C/min et 50°C/s et de préférence 5°C/min à 35°C/min.

Un palier isotherme durant en temps, par exemple, de 0,5 à 20 min est éventuellement appliqué lorsque la température $\theta_3$ est atteinte. Cet isotherme peut avantageusement être appliqué de 1 à 10 minutes.

Les effluents de pyrolyse sont détectés par un détecteur d'hydrocarbures, par exemple un détecteur à ionisation de flamme, et le signal obtenu P est intégré comme précédemment et conservé en mémoire du microprocesseur 5.

La nacelle 1 contenant le résidu de pyrolyse est ensuite transférée par un autre piston 7 et un autre vérin 15 dans un deuxième four 6, avantageusement distinct du premier. On effectue alors une combustion sous atmosphère oxydante grâce à une ligne 13 (air, oxygène ou mélange air-oxygène où le pourcentage d'oxygène représente utilement plus de 20% de la quantité totale) et à une température $\theta_4$ comprise entre 500°C et 1500°C, de préférence entre 600 et 700°C durant un temps compris par exemple entre 2 et 20 minutes et de manière avantageuse entre 5 et 10 minutes.

Le carbone organique présent dans le résidu se transforme en dioxyde de carbone et en monoxyde de carbone et le mélange gazeux passe par une ligne 16 dans un four à catalyse 8 à une température permettant de transformer pratiquement tout le monoxyde de carbone en dioxyde de carbone dans des conditions connues en soi, ce four contenant un catalyseur d'oxydation (CuO par exemple). Le dioxyde de carbone est retenu sur un piège 10 à azote liquide ou à adsorbant solide puis après avoir été libéré du piège par des moyens connus, est détecté par un détecteur spécifique 11, par exemple un catharomètre qui délivre un signal R représentatif de la quantité du résidu.

Ce détecteur 11 peut être suivi en série d'autres détecteurs non représentés sur la figure 1, spécifiques des composés tels qu'oxydes de soufre et/ou d'azote qui auront été piégés pendant l'étape de combustion puis libérés.

La mesure du signal R est conservée en mémoire du microprocesseur 5.

L'intégration du premier signal Q résultant de l'étape de conversion-évaporation permet d'obtenir en milligrammes par gramme par rapport à un étalon la quantité de produits $A_1$, résultant de l'évaporation et de la conversion durant la première étape de chauffage. L'intégration du deuxième signal P résultant de l'étape de pyrolyse permet d'obtenir la quantité d'hydrocarbures lourds pyrolysés $A_2$ en milligrammes par gramme d'échantillon.

Une sone de platine non représentée sur la figure 1 placée dans le four 3 au niveau de l'échantillon permet de mesurer la temperature effective Tmax du sommet de ce pic $A_2$, qui renseigne sur le processus de pyrolyse.

En prenant une valeur moyenne du taux de carbone dans l'échantillon (entre 0,82 et 0,88, par exemple 0,83), il est possible de calculer le taux de carbone organique résultant des étapes d'évaporation $C_Q$ et de pyrolyse $C_P$, par exemple:

$$C_Q = A_1 \times 0,83$$

$$C_P = A_2 \times 0,83$$

D'après le signal R et après étalonnage on en déduit par ailleurs le poids de carbone résiduel $C_R$ détecté sous forme de dioxyde de carbone.

On peut alors calculer le carbone organique total (TOC)

$$TOC = C_Q + C_P + C_R$$

On détermine ainsi la répartition des produits en fractions légère ($F_1$), lourde ($F_2$) et coke ($F_R$) peut être présentée dans un diagramme ternaire, illustré par la figure 2 où sont portés en abscisse $F_R$ et en ordonnée $F_1 + F_2$. Chaque charge pour un catalyseur donné est représentée par un point M avec:

$$F_1 = \frac{100 \times C_Q}{TOC}$$

$$F_2 = \frac{100 \times C_P}{TOC}$$

$$F_R = \frac{100 \times C_R}{TOC}$$

et

$$MQ = F_1 \qquad MP = F_2 \qquad MR = F_R$$

4

Il se vérifie que la fraction légère $F_1$ correspond sensiblement à un distillat et que la fraction lourde $F_2$ correspond sensiblement au domaine des résidus sous-vide.

Le procédé selon l'invention permet d'atteindre les objectifs définis ci-dessus. Il permet en outre à partir d'une charge de référence d'explorer le comportement des catalyseurs de raffinage en isolant les effets d'un paramètre (par exemple l'influence de la teneur en métaux, ou l'influence du support).

Par ailleurs, le procédé est fiable, de mise en oeuvre facile et le temps d'analyse est court (par exemple 30 à 60 minutes).

Les exemples suivants illustrent de manière non limitative l'invention, le premier étant relatif à l'hydrotraitement et le second au craquage catalytique.

Example 1

On considère les catalyseurs A, B, et C d'hydrotraitement de résidus dont les caractéristiques sont reportées au tableau 1, les deux premiers étant des catalyseurs commerciaux et le troisième un catalyseur non commercial.

TABLEAU 1

| Catalyseurs | A procatalyse HMC 845 | B procatalyse HMC 841 | C |
|---|---|---|---|
| Volume poreux total (ml/g) | 0,90 | 0,90 | 0,70 |
| Surface spécifique (m²/g) | 155 | 140 | 300 |
| $MoO_3$% | 7 | 14 | 20,5 |
| NiO % | 1,5 | 3 | 0 |
| CoO % | 0 | 0 | 4,5 |
| $Al_2O_3$ % | 91,5 | 83 | 75 |

Ces catalyseurs A, B et C à l'état neuf, sont soumis à des essais continus d'hydrotraitement d'une charge constituée par du résidu atmosphérique R. A. Safaniya dans les conditions suivantes: T=400°C, VVH=0,5 h$^{-1}$, pression d'hydrogène=120 bars, $H_2$ gazeux/hydrocarbures liquides=1000 normaux litres par litre. La durée de l'essai est d'environ 200 heures. Les caractéristiques de la charge sont indiquées au tableau 2. On mesure au bout de 200 heures selon une technique connue les performances stabilisées de ces catalyseurs en conversion de la charge en fraction 500$^-$ par exemple, qui s'exprime par la relation:

$$\text{conversion } 500^- \text{ (\%)} = 100 \times \frac{(500^+)\text{charge} - (500^+)\text{effluent}}{(500^+)\text{charge}}$$

TABLEAU 2

| | RA Safaniya | RA Koweit |
|---|---|---|
| $d_4^{15}$ (g/cm³) | 0,974 | 0,955 |
| S (% pds) | 4,1 | 3,75 |
| N (ppm) | 2400 | 2000 |
| Asphaltènes $nC_7$ (% poids) | 7 | 2,2 |
| Carbone Conradson (% poids) | 11,9 | 9,2 |
| Viscosité (mm²/s) 80°C | 153 | 53 |
| Ni (ppm) | 26 | 20 |
| V (ppm) | 83 | 48 |

Après ces essais les catalyseurs utilisés sont prélevés, soumis à une extraction en Soxhlet au toluène bouillant pendant 12 heures, séchés à 80°C pendant 6 heures. On détermine ensuite sur ces échantillons le taux de carbone résiduel. On assimile ce carbone, insoluble dans le toluène, à du "coke" catalytique. Les résultats de ces mesures sont détaillés au tableau 3.

TABLEAU 3

| Catalyseur | % conversion 400⁻ | % coke |
|---|---|---|
| A | 41,3 | 12,0 |
| B | 39,7 | 13,8 |
| C | 34,1 | 20,6 |

Parallèlement, d'autres échantillons des catalyseurs A, B et C à l'état neuf sont soumis à des essais de micropyrolyse selon l'invention: ils sont imprégnés au préalable par une solution dans le toluène à 10% poids de résidu atmosphérique R. A. Safaniya.

Le toluène est évaporé sous vide à 80°C pendant 2 heures.

Chaque catalyseur (30 mg) est ensuite broyé et introduit dans une nacelle en acier inoxydable. La nacelle est placée dans un premier four maintenu sous flux d'hélium à un débit de 100 ml/min, à une température de 300°C durant 3 minutes. Les effluents sont détectés par un détection à ionisation de flamme, le signal intégré selon l'invention toutes les 120 secondes et les résultats conservés en mémoire du microprocesseur. On effectue ensuite une montée en température à 30°C/min jusqu'à 600°C. Les effluents de la pyrolyse sont détectés par le détecteur à ionisation de flamme et le signal de pyrolyse intégré toutes les 120 secondes est mis en mémoire; puis on procède à une combustion en plaçant la nacelle contenant l'échantillon dans un deuxième four sous atmosphéric d'air et d'oxygène à un débit de 350 ml/min, à une température de 600°C durant environ 7 minutes. Les oxydes résultant de la combustion, notamment le monoxyde de carbone et le dioxyde de carbone passent ensuite dans un four de catalyse rempli d'oxyde de cuivre et chauffé à environ 400°C pour transformer sensiblement tout le monoxyde de carbone en dioxyde de carbone. Le dioxyde de carbone en totalité est ensuite piégé sur tamis moléculaire de 5 Å ($5 \times 10^{-10}$ m). A la fin de l'étape de combustion, le piège est purgé, et le dioxyde de carbone est détecté par le catharomètre.

Les indices respectifs $F_1$, $F_2$ et $F_R$ calculés selon l'invention sont reportés dans la tableau 4 et sur la figure 2.

TABLEAU 4

| Catalyseur | $F_1$ | | $F_2$ | | $F_R$ | |
|---|---|---|---|---|---|---|
| | R.A. Koweit | R.A. Safaniya | R.A. Koweit | R.A. Safaniya | R.A. Koweit | R.A. Safaniya |
| A | 37,4 | 36,0 | 23,4 | 21,0 | 39,2 | 43,0 |
| B | 26,0 | 23,5 | 25,0 | 22,5 | 49,0 | 54,0 |
| C | 12,0 | 8,0 | 24,5 | 23,0 | 63,5 | 69,0 |

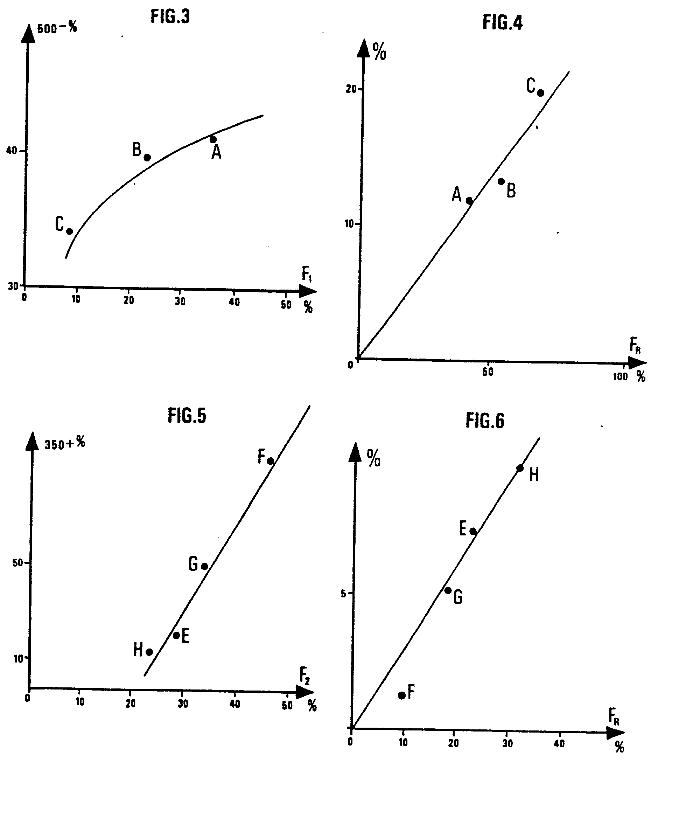

Les figures 3 et 4 illustrent les correlations obtenues pour les catalyseurs d'hydrotraitement A, B et C, entre les résultats selon l'invention et les résultats d'essais de longue durée. Sur la figure 3, sont représentés, en effet, en ordonnée la conversion en fraction 500⁻ mesurée en fin d'essai de longue durée et en abscisse l'indice $F_1$ selon l'invention tandis que sur la figure 4 sont représentés en ordonnée le pourcentage de coke après hydrotraitement déterminé selon la méthode connue de l'art antérieur et l'indice de coke $F_R$ selon l'invention. La bonne corrélation montre la validité du test selon l'invention qui pourra donc être utilisé valablement pour apprécier les activités de conversion et de cokéfaction de catalyseurs différents.

Exemple 2

On reprend l'exemple 1 où l'on remplace la charge Safaniya par un résidu atmosphérique R. A. Koweit dont les caractéristiques sont présentées dans le tableau 2. Le paramètres $F_1$, $F_2$, et $F_R$ selon l'invention sont présentés dans le tableau 4, pour chaque catalyseur A, B et C.

On constate à l'examen du tableau 4 que, lorsque le procédé selon l'invention utilise un résidu atmosphérique de caractéristiques légèrement différentes, le classement obtenu entre les catalyseurs n'est pas affecté. Les corrélations observées restent valables.

Exemple 3

Il est relatif à l'étude de deux catalyseurs de craquage catalytique.

On compare en effet une silice pure F de surface spécifique égale à 200 m²/g et un catalyseur commercial E préalablement soumis à un traitement de 2 heures à 800°C sous 1 atmosphère de vapeur d'eau (DA 250 de Grace Davison). On utilise comme test de référence le test dit M.A.T. (Micro-Activity-Test) selon la norme ASTM D 39-07. La charge des essais M.A.T. est un mélange comprenant 30% de résidu atmosphétique R. A. Kirkouk+70% de distillat sous vide D.S.V. Kirkouk, dont les caractéristiques sont présentées au tableau 5.

TABLEAU 5

| | |
|---|---|
| $d_4^{15}$ | 0,9352 |
| S % | 2,63 |
| Carbone Conradson % | 2,87 |
| Asphaltènes n $C_7$ % | 0,2 |
| Distillation | |
| 5% | 305°C |
| 50% | 465°C |
| 90% | 575°C |

Les résultats du test M.A.T. sont décrits dans le tableau 6.

TABLEAU 6

| Bilan M.A.T. | DA 250 | Silice pure |
|---|---|---|
| conversion (%) | 60,7 | 6,4 |
| gaz totaux (% pds) | 13,4 | 3,6 |
| Essence $C_5$-221°C (% pds) | 39,7 | 2,6 |
| gazole (L.C.O.) 221—350°C (% pds) | 17,9 | 4,7 |
| résidu liquide 350°C (% pds) | 21,2 | 88,8 |
| coke (% pds) | 7,6 | 1,4 |

Par ailleurs, les mêmes catalyseurs sont soumis à un essai selon l'invention. Ils sont imprégnés par une solution de R. A. Koweit à 10% poids dans le toluène.

Le toluène est évaporé 2 heures sous vide primaire à 80°C.

TABLEAU 7

| | DA 250 E | Silice pure F |
|---|---|---|
| $F_1$ (% pds) | 46,3 | 42,6 |
| $F_2$ (% pds) | 29,8 | 47,0 |
| $F_R$ (% pds) | 23,9 | 10,4 |

Les échantillons imprégnés sont soumis à la micropyrolise décrite dans l'exemple 1. Les résultats sont portés dans le tableau 7 et la figure 2 qui précisent les valeurs des indices $F_1$, $F_2$ et $F_R$.

Deux exemples de corrélation sont illustré dans les figures 5 et 6. Afin de préciser la corrélation, on a traité avec la même charge deux autres catalyseurs G et H selon le procédé de l'invention et le test M.A.T. décrits ci-dessus dans ce même exemple. Le catalyseur G est un alumino-silicate tandis que le catalyseur H est le catalyseur commercial DA 250 de Grace Davison dopé par 0,5% poids de nickel. On a donc porté en ordonnée, sur la figure 5, la fraction 350$^+$ obtenue par l'art antérieur (M.A.T.) et en abscisses l'indice $F_2$ obtenu par le procédé selon l'invention. De même, sur la figure 6, on a porté en ordonnée le pourcentage de coke suivant l'art antérieur et en abscisse l'indice de coke $F_R$ selon l'invention.

On note donc la bonne corrélation entre la méthode selon l'invention et le test M.A.T.

## Revendications

1. Procédé de caracatérisation d'un catalyseur par la détermination de ses activités de conversion et de cokéfaction, comprenant une étape de conversion-évaporation et une étape de pyrolyse en atmopshère inerte, caractérisé par les étapes successives suivantes:

a) on imprègne le catalyseur par un mélange d'hydrocarbures,

b) on chauffe sous flux de gaz inerte le catalyseur imprégné jusqu'à une température $\theta_2$ comprise entre 360°C et 380°C de façon à réaliser durant le temps de montée à ladite température ladite étape de conversion-évaporation et l'on un effluent de conversion-évaporation,

c) on mesure sur ledit effluent de conversion-évaporation, au cours du chauffage, au moyen d'un détecteur d'hydrocarbures, un signal Q représentatif des hydrocarbures générés durant ladite étape de conversion-évaporation,

d) on poursuit le chauffage depuis la température $\theta_2$ jusqu'à la température $\theta_3$ comprise entre 500°C et 1000°C de manière progressivement croissante et sous flux de gaz inerte de façon à réaliser durant le temps de montée à ladite température ladite étape de pyrolyse et l'on produit un effluent de pyrolyse et un résidu,

e) on mesure sur ledit effluent de pyrolyse, au cours du chauffage, au moyen d'un détecteur d'hydrocarbures, un signal P représentatif des hydrocarbures pyrolysés générés durant ladite étape de pyrolyse,

f) on réalise ensuite sur ledit résidu une combustion à une température $\theta_4$ comprise entre 500°C et 1500°C sous courant de gaz oxydant, ladite combustion produisant un effluent gazeux de combustion,

g) on mesure sur ledit effluent gazeux de combustion au moyen d'au moins un détecteur approprié, un signal R représentatif de la quantité dudit résidu, et

h) on déduit à partir desdites mesures des signaux P, Q et R les activités de conversion et de cokéfaction dudit catalyseur.

2. Procédé selon la revendication 1 dans lequel on imprègne le catalyseur par le mélange d'hydrocarbures en solution à 1—30% en poids dans un solvant de point d'ébullition inférieur au point d'ébullition initial du mélange d'hydrocarbures et on élimine ensuite le solvant.

3. Procédé selon la revendication 1 ou 2 dans lequel on chauffe le catalyseur de la température ambiante jusqu'à la température $\theta_2$ à une vitesse de programmation de température comprise entre 2°C/mn et 50°C/s.

4. Procédé selon l'une des revendications 1 à 3 dans lequel on chauffe le catalyseur à une vitesse d'augmentation de température de 2°C/mn à 50°C/s durant l'étape de pyrolyse.

5. Procédé selon l'une des revendications 1 à 4 dans lequel on mesure une série de signaux au cours de l'étape de conversion-évaporation, chacun d'une durée de 1 à 120 s, et on intègre ces signaux de manière à obtenir le signal Q.

6. Procédé selon l'une des revendications 1 à 5, dans lequel on mesure une série de signaux au cours de l'étape de pyrolyse, chacun d'une durée de 1 à 120 s, et on intègre ces signaux de manière à obtenir le signal P.

7. Procédé selon l'une des revendications 1 à 6 dans lequel on utilise pour le chauffage en atmosphère inerte un débit spécifique de gaz rapporté à la quantité de matrice poreuse de 1 à 50 millilitres par milligramme et par minute.

8. Procédé selon l'une des revendications 1 à 7 dans lequel on réalise ladite combustion durant 2 à 20 minutes.

9. Procédé selon l'une des revendications 1 à 8 dans lequel ledit détecteur approprié est un détecteur spécifique du dioxyde de carbone tel qu'un catharomètre suivi éventuellement d'un détecteur spécifique des composés soufrés et/ou azotés.

## Patentansprüche

1. Verfahren zur Charakterisierung eines Katalysators durch die Bestimmung seiner Umwandlungs- und Verkokungsaktivitäten, mit einer Umwandlungsverdampfungs- und einer Pyrolysestufe in inerter Atmosphäre, gekennzeichnet durch die folgenden Stufen:

a) man imprägniert den Katalysator mit einem Kohlenwasserstoffgemisch,

b) man erwärmt den imprägnierten Katalysator unter einem Inertgasstrom bis auf eine Tmeperatur $\theta_2$,

die zwischen 300°C und 380°C liegt, derart, daß während der Zeit des Anstiegs auf diese Temperatur diese Umwandlungsverdampfungstufe durchgeführt wird und man einen Umwandlungs-Verdampfungsabstrom erzeugt,

c) man mißt an diesem Umwandlungsverdampfungsabstrom während des Erwärmens vermittels eines Kohlenwasserstoffdetektors ein Signal Q, welches repräsentativ für die während dieser Umwandlungsverdampfungsstufe erzeugten Kohlenwasserstoffe ist,

d) man führt die Erwärmung von der Temperatur $\theta_2$ bis zur Temperatur $\theta_3$ zwischen 500°C und 1000°C in allmählich steigender Weise und unter einem Inertgasstrom derart fort, daß während der Zeit des Anstiegs auf diese Temperatur diese Pyrolysestufe durchgeführt wird und man einen Pyrolyseabstrom und einen Rest erzeugt,

e) man mißt an diesem Pyrolyseabstrom während des Erwärmens vermittels eines Kohlenwasserstoffdetektors ein Signal P, das repräsentativ für die pyrolysierten Kohlenwasserstoffe ist, die während dieser Pyrolysestufe erzeugt wurden,

f) man führt dann an diesem Rest eine Verbrennung bei einer Temperatur $\theta_4$ zwischen 500°C und 1500°C unter einem oxydierenden Gasstrom durch, wobei die Verbrennung einen gasförmigen Verbrennungsabstrom erzeugt,

g) man mißt an diesem gasförmigen Verbrennungsabstrom vermittels wenigstens eines geeigneten Detektors ein Signal R, das repräsentativ für die Menge dieses Restes ist, und

h) man leitet aus diesen Messungen der Signale P, Q und R die Umwandlungs- und Verkokungsaktivitäten dieses Katalysators her.

2. Verfahren nach Anspruch 1, dadurch gekennzeichnet, daß man den Katalysator durch das Kohlenwasserstoffgemisch in Lösung von 1—30 Gew.-% in einem Lösungsmittel mit einem Siedepunkt imprägniert, der niedriger als der Anfangssiedepunkt des Kohlenwasserstoffgemisches liegt und eliminiert dann des Lösungsmittel.

3. Verfahren nach Anspruch 1 oder 2, dadurch gekennzeichnet, daß man den Katalysator von der Umgebungstemperatur bis auf die Temperatur $\theta_2$ bei einer Geschwindigkeit der Temperaturprogrammierung zwischen 2°C/min. und 50°C/sek. erwärmt.

4. Verfahren nach einem der Ansprüche 1 bis 3, dadurch gekennzeichnet, daß man den Katalysator bei einer Geschwindigkeit der Temperaturerhöhung von 2°C/min. auf 50°C/sek. während der Pyrolysestufe erwärmt.

5. Verfahren nach einem der Ansprüche 1 bis 4, dadurch gekennzeichnet, daß man eine Reihe von Signalen während der Umwandlungs-Verdampfungsstufe, je von einer Dauer von 1 bis 120 sek., mißt und daß man diese Signale integriert, derart, daß das Signal Q erhalten wird.

6. Verfahren nach einem der Ansprüche 1 bis 5, dadurch gekennzeichnet, daß man eine Reihe von Signalen während der Pyrolysestufe, je von einer Dauer von 1 bis 120 sek. mißt und daß man diese Signale derart integriert, daß man das Signal P erhält.

7. Verfahren nach einem der Ansprüche 1 bis 6, dadurch gekennzeichnet, daß man zum Erwärmen in inerter Atmosphäre eine spezifische Gasmenge, bezogen auf die Menge an poröser Matrix von 1 bis 50 Milliter pro Milligramm und Minute, verwendet.

8. Verfahren nach einem der Ansprüche 1 bis 7, dadurch gekennzeichnet, daß man diese Verbrennung 2 bis 20 min. lang durchführt.

9. Verfahren nach einem der Ansprüche 1 bis 8, dadurch gekennzeichnet, daß dieser geeignete Detektor ein spezifischer Kohlendioxyddetektor, beispielsweise ein Katharometer, gegebenenfalls gefolgt von einem spezifischen Detektor der schwefelhaltigen und/oder stickstoffhaltigen Verbindungen, ist.

**Claims**

1. Process for characterising a catalyst by determining its conversion and coking activities comprising a conversion evaporation step and a pyrolysis step in an inert atmosphère, caracterised by the following successive steps:

a) impregnating the catalyst with a mixture of hydrocarbons,

b) heating the impregnated catalyst under a flow of inert gas to a temperature $\theta_2$ of between 300°C and 380°C so as to carry out said conversion-evaporation step during the rise time to that temperature, and producing a conversion-evaporation effluent,

c) measuring on said conversion-evaporation effluent in the course of the heating operation, by means of a hydrocarbons detector, a signal Q representative of the hydrocarbons generated during said conversion-evaporation step,

d) continuing the heating from the temperature $\theta_2$ to the temperature $\theta_3$ of between 500°C and 1000°C in a progressively increasing manner and under a flow of inert gas so as to carry out a pyrolysis step during the rise time to said temperature, and producing a pyrolysis effluent and a residue,

e) measuring on said pyrolysis effluent in the course of the heating operation, by means of a hydrocarbons detector, a signal P representative of the pyrolyzed hydrocarbons generated during said pyrolysis step,

f) then subjecting said residue to combustion at a temperature $\theta_4$ of between 500°C and 1500°C under a stream of oxidizing gas, said combustion producing a gaseous combustion effluent,

9

g) measuring on said gaseous combustion effluent by means of at least one suitable detector a signal R representative of the amount of said residue, and

h) deducing from said measurements of the signals Q, P and R the conversion and coking activities of said catalyst.

2. A process according to claim 1 wherein the catalyst is impregnated by the mixture of hydrocarbons in a 1 to 30% solution by weight in a solvent with a boiling point lower than the initial boiling point of the mixture of hydrocarbons and the solvent is then removed.

3. A process according to claim 1 or claim 2 wherein the catalyst is heated from ambient temperature to the temperature $\theta_2$ at a temperature programming speed of between 2°C/min and 50°C/s.

4. A process according to one of claims 1 to 3 wherein the catalyst is heated at a temperature rise speed of 2°C/min to 50°C/s during the pyrolysis step.

5. A process according to one of claims 1 to 4 wherein a series of signals is measured in the course of the conversion-evaporation step, each of a duration of from 1 to 120 s, and said signals are integrated so as to produce the signal Q.

6. A process according to one of claims 1 to 5 wherein a series of signals is measured in the course of the pyrolysis step, each of a duration of from 1 to 120 s, and said signals are integrated so as to produce the signal P.

7. A process according to one of claims 1 to 6 wherein, for the heating operation in an inert atmosphere, there is used a specific flow rate of gas with respect to the amount of porous matrix of from 1 to 50 millilitres per milligram and per minute.

8. A process according to one of claims 1 to 7 wherein said combustion operation is carried on for a period of from 2 to 20 minutes.

9. A process according to one of claims 1 to 8 wherein said suitable detector is a specific detector of the carbon dioxide such as a cathorometer eventually followed by a specific detector of sulphur and/or nitrogen compounds.

**FIG.1**

**FIG.2**

FIG.3

FIG.4

FIG.5

FIG.6